# EUROPEAN PATENT APPLICATION

(11) **EP 2 338 452 A1**
(43) Date of publication of application: **29.06.2011**
(21) Application number: 09180686.9
(22) Date of filing: 23.12.2009
(51) Int. Cl.: A61F 13/56

(54) **Hygiene Article**

(71) Applicant: 3M Innovative Properties Company, Saint Paul, MN 55133-3427 (US)
(72) Inventor: Petersen, Johann, 41516 Grevenbroich (DE)
(74) Representative: Wilhelm, Stefan

(57) **Abstract**

A hygiene article employing adhesive fastening means, particularly an adult incontinence pad, which does not require the use of a separate release liner, is disclosed. The hygiene article comprises an elongate absorbent core sandwiched between a back sheet and a front sheet, with the back sheet and the front sheet extending sufficiently beyond the absorbent core to form a sealing edge region adjacent the absorbent core. At least one adhesive fastening means is positioned on or adjacent to the sealing edge region and being covered with an adjacent release surface. At least part of the release surface is carried on a portion of the sealing edge region. Preferably the release means is removable from the hygiene article.

## Description

The present invention relates to a hygiene article, in particular, a hygiene article having an elongate absorbent core sandwiched between a back sheet and a front sheet, suitable for use as a disposable adult incontinence pad.

Hygiene articles, particularly disposable hygiene articles have a variety of uses, including infant diapers (sometimes also known as nappies), feminine hygiene articles (such as sanitary towels or napkins and panty liners), and adult incontinence wear (for example, incontinence pads and disposable undergarments). Each of these articles is designed to absorb and/or retain liquids and other body exudates and has in common the need for the article to be fixed in position relative to the body of a wearer. This may be done by adhering the article directly to itself such that it surrounds a wearer (in the case of infant diapers and disposable undergarments) or to an item of clothing (in the case of feminine hygiene articles and adult incontinence pads), or directly to the skin of a user (in the case of feminine hygiene articles and incontinence pads). One of the most popular fixation mechanisms is to adhere the hygiene article to an item of clothing, for example, an undergarment. In general, there are two main ways in which this may be achieved (a) using adhesive fastening means or (b) using mechanical fastening means. Whilst a mechanical fastening means offers advantages with regard to the removability of the hygiene article after use, adhesive fastening means remain extremely popular with manufacturers and users alike, due to their low cost, versatility and ease of use.

However, in order to ensure that an adhesive is not damaged or contaminated during storage or transport, it is necessary to protect the exposed surface of the adhesive to prevent unwanted adhesion or adhesive transfer. This is commonly achieved using a release liner covering the adhesive region that is removed just before the hygiene article is positioned in contact with a wearers' clothing, or by providing the hygiene article within removable packaging that acts as a release liner for any adhesive regions on the hygiene article, which are exposed when the hygiene article is removed from the packaging. Whilst such liners are undoubtedly extremely useful, they require not only additional materials to those used in the hygiene article construction, but additional manufacturing steps in order to be applied to the hygiene article before packing. Alternative approaches include using an adhesive that does not require the use of a release liner or, in the case of a sanitary towels or napkins having side flaps or "wings", using a portion of this additional material as a release liner.

An example of the use of securing flaps or "wings" as a release liner for adhesive regions on a sanitary towel or napkin is disclosed in US 5, 472, 437. The sanitary napkin comprises a main napkin body and is provided with side flaps extending from the main napkin body. The flaps are provided prevent the lateral leakage of body fluids from the main body of the napkin, and consequently require adhesion to the back side of the edge of a users' undergarment into which the napkin is fixed. This adhesion is provided by a region of adhesive material positioned on the back side of the side flaps, with the main body of the napkin being adhered to the undergarment by a strip of adhesive material positioned on the back side of the main napkin body. As an alternative to a release liner, a portion of the back side of the side flap is used to cover the regions of adhesive material when not in use. The release liner function can be provided by using a release agent, such as a silicone or a urethane release agent, or by providing a region of an adhesive that is incompatible with and non-adhesive to the adhesive used to affix the flap.

This design relies on the fact that a typical sanitary napkin design comprises side flaps formed from additional material to the main body of the sanitary napkin, and consequently there is a reasonable area of material that is useable as a release liner. However, not all designs of hygiene article, such as adult incontinence pads, incorporate such side flaps. This may be for several reasons, for example, the additional cost in providing excess material and associated wastage when shaped napkin designs are cut from long rectangular rolled sheets of materials used to form the front sheet and back sheet of the hygiene article, or that there is no requirement for a side flap to prevent leakage of bodily fluids as the fluids are incident on a different portion of the hygiene article, which, when in use, does not correspond to the gusset portion of an undergarment where such flaps may be positioned in contact with the back side of the undergarment easily. In an adult incontinence pad, for example, it may be desirable for the pad to have larger regions at each end, corresponding to the front and the rear of the pad when worn, and a narrower central region corresponding to a gusset, in a similar shape to an infant diaper, to allow for easier movement of the legs of the wearer. In a low-cost hygiene article, comprising a front sheet and a back sheet surrounding an absorbent core and having a basic rectangular shape, there is no excess material provided in order to keep manufacturing costs to a minimum. In both of these situations there is no choice other than using a separate release liner to protect adhesive regions before use.

It is desirable therefore to find an alternative to a separate release liner in hygiene article designs that do not incorporate side flaps or other regions of excess material.

The present invention aims to address these problems by providing a hygiene article, comprising: an elongate absorbent core sandwiched between a back sheet and a front sheet, the back sheet and the front sheet extending sufficiently beyond the absorbent core to form a sealing edge region adjacent the absorbent core; and at least one adhesive fastening means positioned on or adjacent and proximate to the sealing edge region and being covered with a release surface positioned adjacent to the adhesive fastening means, wherein at least part of the release surface is carried on a portion of the sealing edge region.

By providing the adhesive fastening means and associated release surface on or adjacent to the sealing edge it is possible to avoid the use of separate release liners, as the hygiene article itself acts as a release liner to the adhesive fastening means positioned thereon. It is particularly advantageous to have both the adhesive fastening means and the release surface positioned on and carried by the sealing edge as the release surface may be provided on a region of the sealing edge that would otherwise be discarded during the manufacturing process, and therefore creates a disposable release liner. By having the release surface positioned adjacent to the adhesive fastening means, that is, neighbouring the adhesive fastening means in close proximity, the amount of material required to carry the release surface as a portion of the hygiene article is minimised. The overall shape of the hygiene article remains the same as known products, corresponding to the sizes known and preferred by a user.

Preferably substantially all of the release surface is carried on the sealing edge region.

The hygiene article may comprise a fold line between the adhesive fastening means and the corresponding release surface. In this situation, the release surface may be detachable from the hygiene article, and/or perforations may be provided along the fold line. By making the release surfaces disposable they may be removed before the hygiene article is used, in a similar manner to existing products with separate liners, which may be favourable for a user. In addition, by placing the release surfaces on a portion of the sealing edge that may be disposed of, no substantial redesigning of the shape and/or size is required to provide an available surface to carry the release liner.

The hygiene article may have an elongate shape comprising first and second opposing longitudinal sides that is, the long sides of a substantially rectangular elongate shape) and first and second opposing transverse sides (that is, the short sides of a substantially rectangular elongate shape), with least one adhesive fastening means being provided on each opposing longitudinal side.

The hygiene article may have an elongate shape comprising first and second opposing longitudinal sides (that is, the long sides of a substantially rectangular elongate shape) and first and second opposing transverse sides (that is, the short sides of a substantially rectangular elongate shape) with at least one adhesive fastening means being provided on each opposing transverse side.

Preferably, the adhesive fastening means are provided on the back sheet. This is an advantageous construction as it enables the hygiene article to be adhered directly to the clothing of a wearer. Alternatively, the adhesive fastening means may be provided on the front sheet. For example, an adhesive compatible with a wearers' skin may be used.

Adhesive fastening means may also be provided in the corners defined by the intersections of the first and second opposing longitudinal sides with the first and second opposing transverse sides. This gives increased adhesion to an undergarment at corners of the hygiene article without increasing the overall size of the article by needing to provide side or end flaps to mount an adhesive fastening means.

The first and second opposing longitudinal sides may comprise cut-out regions along the sealing edge each adapted to accommodate the leg of a wearer, with at least one adhesive fastening means being provided adjacent to the cut-out regions. The portion of the sealing edge on which the release surface is carried is preferably the selvedge of the cut-out regions. This approach allows material that would otherwise be wasted to be used as a release surface.

Preferably, the release surface is formed from a low-adhesion backsize coating. Such a coating is particularly suitable for use with the pressure sensitive adhesives used in personal hygiene applications, and offers an excellent alternative to additional release liners.

The present invention also provides for the use of a hygiene article as described above as an adult incontinence pad.

The invention will now be described by way of example only, and with reference to the accompanying drawings, in which:
Figure 1 is a diagrammatic plan view of a typical hygiene article, showing the shape of the article and the initial outline of the article during manufacture;
Figure 2 is a diagrammatic cross-section of the hygiene article of Figure 1, showing the layer construction of the article;
Figure 3 is a diagrammatic plan view of a hygiene article in accordance with a first embodiment of the present invention;
Figure 4 is a diagrammatic plan view of the hygiene article of Figure 3 illustrating the folded regions of the sealing edge;
Figure 5 is a diagrammatic plan view of a hygiene article in accordance with a second embodiment of the present;
Figure 6 is a diagrammatic plan view of the hygiene article of Figure 5 illustrating the folded regions of the sealing edge;
Figure 7 is a diagrammatic plan view showing a first manufacturing stage of a hygiene article in accordance with a first embodiment of the present invention;
Figure 8 is a diagrammatic plan view showing a second manufacturing stage of a hygiene article in accordance with a first embodiment of the present invention;
Figure 9 is a diagrammatic plan view showing a third manufacturing stage of a hygiene article in accordance with a first embodiment of the present invention; and
Figure 10 is a diagrammatic plan view showing a fourth manufacturing stage of a hygiene article in accordance with a first embodiment of the present invention.

Rather than providing a separate release liner or any regions of excess material that could function as a release liner for an adhesive provided on a surface of a hygiene article, the present invention takes the approach that the sealing edge formed around the periphery of a hygiene article when a front sheet and a back sheet are joined together to contain an elongate absorbent core can be used to carry a release surface, or the release surface may be positioned adjacent and proximate to this sealing edge. This release surface may be positioned adjacent the adhesive fastening means it is intended to protect, and may be formed from an area of material that is conventionally discarded as selvedge in the manufacture of hygiene articles. In this manner, the release surface may remain attached to the hygiene article when the article is in use, or be disposed of once removed from the adhesive fastening means it protects. As the shape and position of the adhesive fastening means provided on the hygiene article may vary, the adoption of the sealing edge to carry the release surface results in design flexibility, ease of manufacture (as no complex shapes must be cut from lengths of material running through a production line) and reduced manufacturing costs (as little or no material is wasted in the construction of the hygiene article). The hygiene article itself is preferably an adult incontinence pad, which typically are not provided with edge flaps or other regions of excess material, and are generally elongate with a substantially rectangular periphery or else are shaped so as to provide a cut-out region providing ease of movement for a wearer's legs. However, the present invention is also suitable for use with feminine hygiene articles that do not employ edge flaps or other extensions, for example, panty liners.

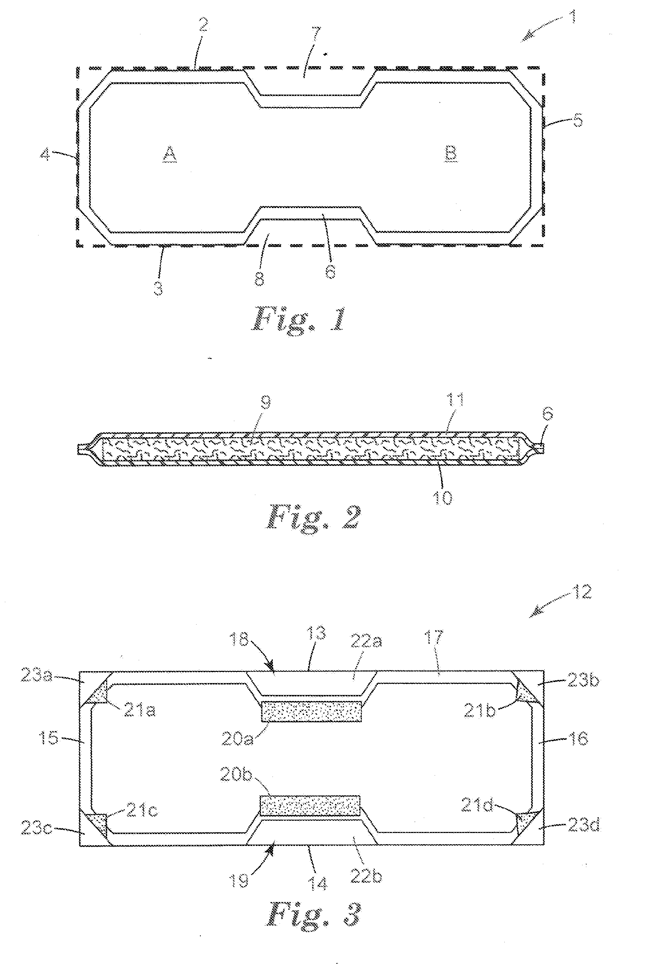

Figure 1 is a diagrammatic plan view of a typical hygiene article, showing the shape of the article and the initial outline of the article during manufacture. The hygiene article 1 is generally elongate in shape, comprising first 2 and second 3 opposing longitudinal sides and first 4 and second 5 opposing transverse sides. The hygiene article 1 comprises an absorbent core 9 sandwiched between a back sheet 10 and a front sheet 11 (see Figure 2), where the back sheet and the front sheet extend beyond the absorbent core to form a sealing edge region 6 adjacent the absorbent core. This sealing edge 6 forms the periphery of the hygiene article 1. The first 2 and second 3 longitudinal sides comprise cut-out regions 7, 8 along the sealing edge 6, each adapted to accommodate the leg of a wearer. This creates two regions A, B adjacent the opposing transverse sides 4, 5 of the hygiene article 1 that are slightly larger in width than the central region defined by the position of the cut-out regions 7, 8, which will form the front and rear of the hygiene article 1 when worn in use. Also shown (as a dashed line) is the extent to which the back sheet and the front sheet extend to create the initial outline of the article during manufacture. As can be seen in Figure 1, this initial extension creates regions that require removal as selvedge. The initial outline during manufacture is indicated as a bold dashed line around the periphery of the hygiene article 1.

Figure 2 is a diagrammatic cross-section of the hygiene article of Figure 1, showing the layer construction of the article. As described above, the hygiene article comprises an elongate absorbent core 9, formed from a compressible, conformable material capable of absorbing and/or retaining liquids and other body exudates. Suitable materials include comminuted wood pulp (airfelt) creped cellulose wadding, absorbent foams, absorbent sponges, superabsorbent polymers, absorbent gelling materials or a combination of any of these. The absorbent core 9 may be substantially rectangular in shape, or may be shaped to follow the outline of the hygiene article, that is, comprise cut-out regions corresponding to the leg position of a wearer. These cut-out regions may have gathered edges, for example, to provide a stretchable region, or may be plain. The absorbent core 9 is sandwiched between a back sheet 10 and a front sheet 11 that extend sufficiently beyond the absorbent core 9 to form the sealing edge 6. The back sheet 10 may be made from a flexible liquid impermeable material such as a thin plastic film, for example, polyethylene film, having a thickness in the range of 0.01mm to 0.05mm. The top sheet 11 may be formed from a liquid permeable material such as a porous foam, a porous film, or a woven or non-woven fabric of natural (for example wood or cotton) and/or synthetic (for example polyester or polyethylene) fibres. If desired, the top sheet 11 may also have hydrophobic properties to prevent the wearers' skin from coming into contact with liquids or other body exudates. The sealing edge 6 may be formed by bonding the back sheet 10 and the front sheet 11 together using an adhesive or other bonding process, such as thermal bonding or ultrasonic welding. The sealing edge 6 extends beyond the absorbent core 9 sufficiently to ensure that the absorbent core 9 is held firmly in position and is covered completely by the back sheet 10 and the front sheet 11. If desired, the absorbent core 9 can be bonded to the back sheet 10 using an adhesive, or can be held in position between the back sheet 10 and the front sheet 11 purely by the presence of the sealing edge 6.

Figure 3 is a diagrammatic plan view of a hygiene article in accordance with a first embodiment of the present invention. The hygiene article 12 has the same general construction as illustrated in Figures 1 and 2, that is it is generally elongate in shape, comprising first 13 and second 14 opposing longitudinal sides and first 15 and second 16 opposing transverse sides. The hygiene article 12 comprises an absorbent core sandwiched between a back sheet and a front sheet, where the back sheet and the front sheet extend beyond the absorbent core to form a sealing edge region 17 adjacent the absorbent core. This sealing edge 17 forms the periphery of the hygiene article 12. The first 13 and second 14 longitudinal sides comprise cut-out regions 18, 19 along the sealing edge 17, each adapted to accommodate the leg of a wearer. The hygiene article 12 is provided with an adhesive fastening means 20a, 20b on each opposing longitudinal side 13, 14. Each adhesive fastening means 20a, 20b is provided adjacent the cut-out region 18, 19 in each opposing longitudinal side 13, 14. These adhesive fastening means 20a, 20b are substantially rectangular in shape, and of approximately the same length as the cut-out region 18, 19 to which it is adjacent. The hygiene article 12 is also provided with adhesive fastening means 21 a, 21 b, 21c, 21 d in the corners defined by the intersections of the first 13 and second 14 opposing longitudinal sides with the first 15 and second 16 opposing transverse sides. Each of these adhesive fastening means 21a, 21b, 21c, 21 d is substantially triangular in shape. In this embodiment, each adhesive fastening means 20a, 20b, 21a, 21b, 21c, 21 d is positioned on the back sheet of the hygiene article 12, such that it can contact the undergarment of a wearer to hold the hygiene article 12 firmly in place when being worn in use.

A release surface is provided on the sealing edge 17 for each of the adhesive fastening means 20a, 20b, 21a, 21b, 21c, 21 d. The release surfaces are provided adjacent to the respective adhesive fastening means. A release surface 22a, 22b is provided in each cut-out region 18, 19. These are carried on the selvedge of the cut-out regions 18, 19, that is, the material that would be removed as waste from the conventional hygiene article 1 design shown in Figure 1. A release surface 23a, 23b, 23c, 23d is provided for each adhesive fastening means 21a, 21b, 21c, 21d in the corners defined by the intersections of the first 13 and second 14 opposing longitudinal sides with the first 15 and second 16 opposing transverse sides. Each of these release surfaces is carried on the selvedge of the intersection regions, that is, the material that would be removed as waste from the conventional hygiene article 1 design show in Figure 1. Regardless of its position, each release surface 22a, 22b, 23a, 23b, 23c, 23d, is preferably provided adjacent the adhesive fastening means 20a, 20b, 21a, 21b, 21c, 21d it is intended to protect. In addition, each release surface 22a, 22b, 23a, 23b, 23c, 23d is preferably substantially the same shape as the adhesive fastening means 20a, 20b, 21a, 21b, 21c, 21d it is intended to protect, and is marginally larger in size than the corresponding adhesive fastening 20a, 20b, 21a, 21b, 21c, 21d, not only to aid in automated processing but to make removal of the release surface 22a, 22b, 23a, 23b, 23c, 23d easier. The first embodiment of the present invention is particularly advantageous as it allows the release surface to be formed from regions of the hygiene article that may otherwise be disposed of as waste. Although this embodiment illustrates the use of adhesive fastening means provided in the corners of the hygiene article, these may be omitted if desired, and are an optional addition to the adhesive fastening means provided in the cut-out regions.

Preferably, the adhesive used is a pressure sensitive adhesive. It may be provided in the form of an adhesive strip or tape bonded to the back sheet 11, or may be a hot-melt spray adhesive. The release surface is preferably a low-adhesion backsize coating (known as LAB coatings), such as, but not limited to, those disclosed in WO99/011683 or US 5,571,586, to which reference should be made.

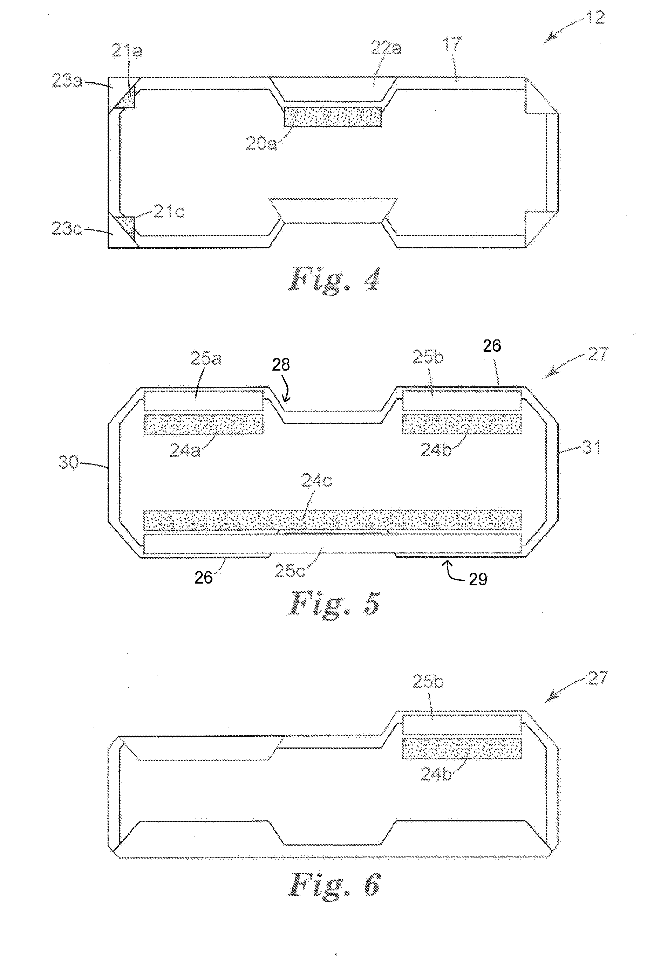

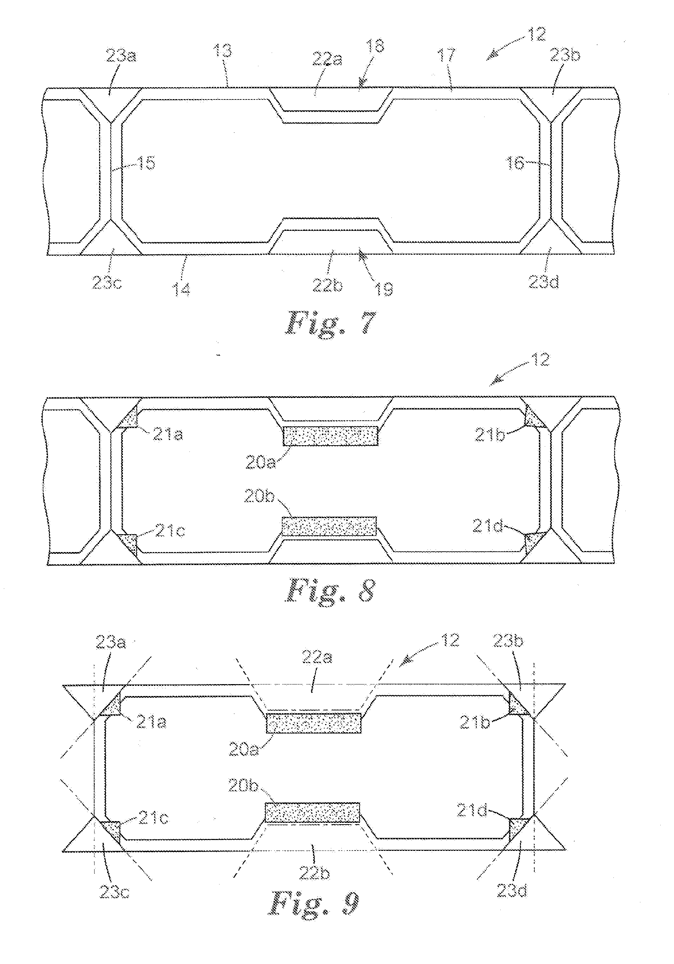

Figure 4 is a diagrammatic plan view of the hygiene article of Figure 3 illustrating the folded regions of the sealing edge. Each of the release surfaces 23a, 23b, 23c, 23d described above is provided next to a fold line (not shown) that separates the release surface 23a, 23b, 23c, 23d from the adhesive fastening means 21 a, 21 b, 21 c, 21 d it is intended to protect. This fold line enables the release surface 23a, 23b, 23c, 23d to be folded over onto the adhesive fastening means 21a, 21b, 21c, 21d it is intended to protect easily, in particular, during mechanical processing on a production line, as described in more detail with reference to Figures 7 to 10 below. In this embodiment, the fold line is provided with perforations (not shown) along its length. This ensures that the release surface 23a, 23b, 23c, 23d is detachable from the hygiene article 21, such that it may be disposed of when the hygiene article 21 is worn in use. When folded over (as shown in Figure 4), the release surface 22b, 23b, 23d covers the adhesive fastening means 20b, 21 b, 21 d it is intended to protect.

In order for the release surfaces 22a 22b to cover the adhesive fastening means 20a, 20b provided in the cut-out regions 18,19, two cut lines are provided in place of perforations (not shown). These cut lines separate the portion of the sealing edge 17 carrying the release surface 22a, 22b from the main body of the hygiene article 12, and are provided along the lines that indicate where excess material would be removed as waste, that is, along the inward diagonal lines defining the depth of the cut-out regions 18, 19. The boundary of the innermost extent of the cut-out regions 18, 19 is provided with a fold line, along which perforations are provided, to allow the release surface 22a, 22b to be folded over onto the adhesive fastening means 20a, 20b it is intended to protect. Perforations are provided such that the release surface 22a, 22b is detachable from the main body of the hygiene article 12 when it is worn in use.

Figure 5 is a diagrammatic plan view of a hygiene article in accordance with a second embodiment of the present invention. This second embodiment illustrates the use of alternative locations for the adhesive fastening means and release surfaces, in that they may be adjacent and proximate to the sealing edge, rather than carried on the sealing edge of the hygiene article. The components of the hygiene article (absorbent core, back sheet, front sheet, adhesive and release surfaces) are the same as those described above in the first embodiment of the present invention. Each of these alternative adhesive fastening means 24a, 24b, 24c and release surface 25a, 25b, 25c locations employs a adhesive fastening means positioned adjacent to the sealing edge 26 of the hygiene article 27. At least a part of the release surface 25a, 25b, 25c is carried on the sealing edge, in contrast to the first embodiment of the present invention where substantially all of the release surface is carried on the sealing edge. For example, along the upper longitudinal side 28 of the hygiene article 27, two adhesive fastening means 24a, 24b are provided. Each of these regions is substantially rectangular in shape, and extends along parallel to a greater portion of the sealing edge 26 between the first 30 and second 31 opposing transverse sides. Each is provided with a release surface 25a, 25b, also substantially rectangular in shape, positioned adjacent to and marginally larger in size than the adhesive fastening means 24a, 24b it is intended to protect. Again, both the adhesive fastening means and the release surfaces are provided on the back sheet of the hygiene article.

Along the lower longitudinal side 29 of the hygiene article 27, a single adhesive fastening means 24c is provided, extending along substantially the entire length the lower longitudinal side 29, parallel to and adjacent to the sealing edge 26. The release surface 25c is provided adjacent to the adhesive fastening means 24c, and again, is substantially rectangular in shape and marginally larger than the adhesive fastening means 24c it is intended to protect. In this alternative configuration, the central part of the release surface 25c is carried on the sealing edge 26, where the sealing edge defines the innermost extent of the cut-out regions designed to accommodate the legs of a wearer. The remainder of the release surface 25c is carried on the main body of the hygiene article 27, adjacent the sealing edge and parallel to it.

Figure 6 is a diagrammatic plan view of the hygiene article of Figure 5 illustrating the folded regions of the sealing edge. To cover each adhesive fastening means 24a, 24c, the sealing edge 27 is folded over such that the release surfaces 25a, 25c contact the adhesive fastening means 24a, 24c they are intended to protect.

It will be appreciated that there are a number of variations in size, shape and position of both adhesive regions and release surfaces that may be employed in accordance with the present invention. For example, although in the above embodiment adhesive fastening means are positioned on the opposing longitudinal sides of the hygiene article, they may be positioned alternatively along the opposing transverse sides of the hygiene article. Although the release surface may be positioned between the adhesive fastening means it is intended to protect and the periphery of the hygiene article, this positioning may be reversed, with the adhesive fastening means positioned between the release surface and the periphery of the hygiene article. Whilst the adhesive fastening means provided along the longitudinal sides of the hygiene article are shown as being substantially rectangular in shape, other shapes may be used instead, for example, ellipsoidal, or small, discrete adhesive fastening means, such as adhesive spots having a variety of shapes, may be used instead of a single rectangular strip of adhesive.

In the above embodiments, the adhesive fastening means and the release surfaces are provided on the back sheet of the hygiene article. However, an alternative is to provide the adhesive fastening means and the release surfaces on the front sheet of the hygiene article, again carried by or adjacent and proximate to the sealing edge. In this situation, an adhesive suitable for adhering hygiene articles to the skin of a wearer, as known in the art, may be used. This allows the hygiene article to be affixed directly to the skin of a wearer as opposed to an undergarment or other item of clothing.

A method of manufacturing a hygiene article in accordance with the first embodiment of the present invention will now be described. It will be clear that these stages are also used in the manufacture of hygiene articles in accordance with the second embodiment of the present invention, and are easily modified to produce the alternative adhesive and release surface configurations described above.

Figure 7 is a diagrammatic plan view showing a first manufacturing stage of a hygiene article in accordance with a first embodiment of the present invention. The hygiene article 12 is shown in outline indicating the positioning of the first 13 and second 14 opposing longitudinal sides, the first 15 and second 16 opposing transverse sides, the position of the cut-out regions 18, 19 and the sealing edge 17 around the periphery of the hygiene article 12. These are shown with respect to a continuous layered hygiene article construction having a back sheet 10, a front sheet 11 and an absorbent core 9, with individual hygiene articles 12 positioned in an end-to-end fashion to minimise wastage. This initial outline is created by providing the sealing edge 17, which may be done using a variety of techniques, including bonding and welding. This may be done on the same production line as the following manufacturing stages, in which case initially separate rolls of back sheet, front sheet and absorbent core material are fed onto a roller system, aligned and the sealing edge 17 formed, or separately to the production line for the following manufacturing stages, such that a roll of pre-formed hygiene article 12 shapes are fed onto a roller system. The hygiene article 12 is shown with the back sheet 10 uppermost.

The first stage is to apply the low-adhesive backsize (LAB) coating forming the release areas 22a, 22b, 23a, 23b, 23c, 23d on the sealing edge 17, The LAB coating is applied in the cut-out regions 18, 19 and at the intersections of the opposing longitudinal 13, 14 and opposing transverse 15, 16 sides. This may be done using known spray technologies, either whilst the hygiene article 12 is held in a stationary position or whilst it is moving along the production line. If the chosen LAB is a curable LAB such as a UV curable LAB as those disclosed in WO 00/31203 an additional step of exposing the release areas 22a, 22b, 23a, 23b, 23c, 23d to a UV light source until the LAB is cured is included in this stage.

Figure 8 is a diagrammatic plan view showing a second manufacturing stage of a hygiene article in accordance with a first embodiment of the present invention. The first stage is to apply the low-adhesive backsize (LAB) coating forming the release areas 22a, 22b, 23a, 23b, 23c, 23d on the sealing edge 17, The LAB coating is applied in the cut-out regions 18, 19 and at the intersections of the opposing longitudinal 13, 14 and opposing transverse 15, 16 sides. This may be done using known coating technologies such as spray coating or printing,, either whilst the hygiene article 12 is held in a stationary position or whilst it is moving along the production line.

Figure 9 is a diagrammatic plan view showing a third manufacturing stage of a hygiene article in accordance with a first embodiment of the present invention. During this stage the adhesive fastening means 20a, 20b, 21 a, 21b, 21c, 21 d are added. This may be done using a spray technology system, for example, if the adhesive chosen is a sprayable hot-melt adhesive, or by positioning strips of adhesive material with an adhesive backing onto the back sheet 10. Again, this may be done whilst the hygiene article 12 is held in a stationary position, or whilst it is moving along the production line. If the chosen adhesive is an ultra violet (UV) curable adhesive an additional step of exposing the adhesive fastening means 20a, 20b, 31 a, 21b, 21c, 21d (and the hygiene article 12) to a UV light source until the adhesive is cured is included during this manufacturing stage.

Also during this stage foldlines and perforations (indicated by a dot-dashed line) are provided between the adhesive fastening means 20a, 20b, 21a, 21b, 21c, 21d and the release surfaces 22a, 22b, 23a, 23b, 23c, 23d formed by the LAB coating. Cutlines (indicated by a dashed line) are also provided between the individual hygiene articles 12 on the continuous layered construction, separating them from each other, and on the inward diagonal foldlines of the cut-out regions 18, 19. This is done using a mechanical scoring device to provide the fold lines and a mechanical cutter to provide both the perforations and the cut lines.

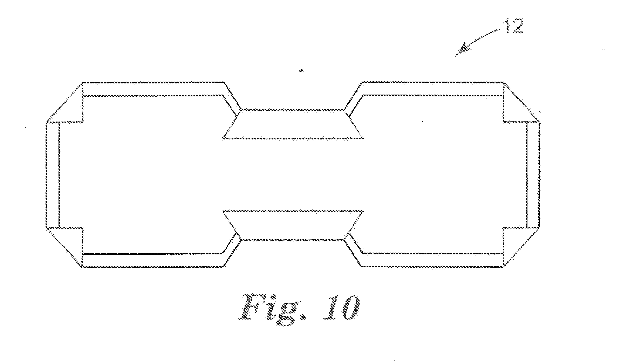

Figure 10 is a diagrammatic plan view showing a fourth manufacturing stage of a hygiene article in accordance with a first embodiment of the present invention. At this point, once the hygiene articles 12 have been separated the LAB coated release surfaces 22a, 22b, 23a, 23b, 23c, 23d are folded over the adhesive fastening means 20a, 20b, 21a, 21b, 21c, 21d using an automated mechanical folding device. Once the adhesive fastening means 20a, 20b, 21a, 21b, 21c, 21d are fully protected the hygiene article 12 may be folded lengthways (for example, by bi-folding with a fold positioned centrally along the opposing longitudinal sides 13, 14, or by tri-folding with two folds positioned along the opposing longitudinal sides 13, 14, as shown, for example, in WO94/04111) for packaging, as is common for such products.

Although the manufacturing process above is described in terms of sprayable coatings, adhesive strips and mechanical scoring, cutting and folding devices, other suitable technologies may be substituted. For example, a laser cutting process may be used in place of a mechanical cutting process, and other adhesive coating methods may be used to replace a sprayable or strip adhesive material.

## Claims

1. A hygiene article, comprising:
an elongate absorbent core sandwiched between a back sheet and a front sheet, the back sheet and the front sheet extending sufficiently beyond the absorbent core to form a sealing edge region adjacent the absorbent core; and
at least one adhesive fastening means positioned on or adjacent and proximate to the sealing edge region and being covered with a release surface positioned adjacent to the adhesive fastening means;
wherein at least part of the release surface is carried on a portion of the sealing edge region.

2. A hygiene article according to claim 1, wherein substantially all of the release surface is carried on the sealing edge region.

3. A hygiene article according to claim 1 or 2, comprising a fold line between the adhesive fastening means and the corresponding release surface.

4. A hygiene article according to claim 3, wherein the release surface is detachable from the hygiene article.

5. A hygiene article according to claim 4, wherein perforations are provided along the fold line.

6. A hygiene article according to any preceding claim, wherein the article has an elongate shape comprising first and second opposing longitudinal sides and first and second opposing transverse sides, and wherein at least one adhesive fastening means is provided on each opposing longitudinal side.

7. A hygiene article according to any preceding claim, wherein the article has an elongate shape comprising first and second opposing longitudinal sides and first and second opposing transverse sides, and wherein at least one adhesive fastening means is provided on each opposing transverse side.

8. A hygiene article according to any preceding claim, wherein the adhesive fastening means are provided on the back sheet.

9. A hygiene article according to any of claims 1 to 7, wherein the adhesive fastening means are provided on the front sheet.

10. A hygiene article according to claim 6 or 7, wherein adhesive fastening means are also provided in the corners defined by the intersections of the first and second opposing longitudinal sides with the first and second opposing transverse sides.

11. A hygiene article according to any of claims 6 to 10, wherein the first and second opposing longitudinal sides comprise cut-out regions along the sealing edge each adapted to accommodate the leg of a wearer, and wherein at least one adhesive fastening means is provided adjacent to the cut-out regions.

12. A hygiene article according to claim 11, wherein the portion of the sealing edge on which the release surface is carried on the selvedge of the cut-out regions.

13. A hygiene article according to any preceding claim, wherein the release surface is formed from a low-adhesion backsize coating.

14. Use of a hygiene article according to any of claims 1 to 13 as an adult incontinence pad.
